# EUROPEAN PATENT APPLICATION

(11) **EP 1 374 772 A1**
(43) Date of publication of application: **02.01.2004**
(21) Application number: 03076570.5
(22) Date of filing: 23.05.2003
(51) Int. Cl.: A61B 5/15, A61M 5/32

(54) **Manual safety device for a medical needle**

(30) Priority: 23.05.2002 US 382745 P; 21.05.2003 US
(71) Applicant: Becton, Dickinson and Company, Franklin Lakes, New Jersey 07417 (US)
(72) Inventor: Swenson, Kirk D., North Caldwell, NJ 07006 (US)
(74) Representative: 't Jong, Bastiaan Jacobus

(57) **Abstract**

A shieldable blood collection set includes a needle cannula and a tip guard axially movable along the needle cannula through a guard drive. The tip guard is axially movable along the needle cannula from a proximal position substantially adjacent a proximal end of the needle cannula, to a distal position in which the tip guard protectively surrounds a puncture tip at the distal end of the needle cannula. The guard drive interconnects the needle cannula and the tip guard through a hub which includes a rigid fin and a tether. The rigid fin is pivotally connected to the hub and extends dorsally from the hub, and the tether interconnects the end of the fin with the tip guard. Pivotal movement of the fin causes the tether to extend to move the tip guard from the proximal position to the distal position for shielding of the needle.

## Description

### BACKGROUND OF THE INVENTION

### 1. Field of the Invention

The present invention relates to blood collection sets for safe and convenient handling of needles. More particularly, the present invention relates to a low cost disposable blood collection set including a needle assembly having a safety shield.

### 2. Description of Related Art

Disposable medical devices having piercing elements are typically used for administering a medication or withdrawing a fluid, such as blood collecting needles, fluid handling needles and assemblies thereof. Current medical practice requires that the fluid containers and needle assemblies used in such systems be inexpensive and readily disposable. Consequently, existing blood collection systems, for example, typically employ some form of durable, reusable holder on which detachable and disposable needles and fluid collection tubes may be mounted. A blood collection system of this nature can be assembled prior to use and then disassembled after usage. Thus, these blood collection systems allow repeated use of the relatively expensive holder upon replacement of the relatively inexpensive needle and/or fluid collection tube. In addition to reducing the cost of collecting blood specimens, these blood collection systems also help minimize the production of hazardous medical waste.

A blood collection set or intravenous (IV) infusion set typically includes a needle cannula having a proximal end, a pointed distal end and a lumen extending therebetween. The proximal end of the needle cannula is securely mounted in a plastic hub with a central passage that communicates with the lumen through the needle cannula. A thin flexible thermoplastic tube is connected to the hub and communicates with the lumen of the needle cannula. The end of the plastic tube remote from the needle cannula may include a fixture for connecting the needle cannula to a blood collection tube or some other receptacle. The specific construction of the fixture will depend upon the characteristics of the receptacle to which the fixture will be connected.

In order to reduce the risk of incurring an accidental needle-stick wound, protection of used needle tips becomes important. With concern about infection and transmission of diseases, methods and devices to enclose the used disposable needle have become very important and in great demand. For example, needle assemblies commonly employ a safety shield that can be moved into shielding engagement with a used needle cannula without risking an accidental needle stick.

Some needle shields are referred to as tip guards, and include a small rigid guard that can be telescoped along the length of a needle cannula and extended over the puncture tip of the needle for protection. Such conventional tip guard may include some form of tether for limiting the travel of the tip guard to the length of the needle cannula. Additionally, such conventional tip guard typically includes a structure that lockingly engages over the tip of the used needle cannula to prevent a re-exposure of the needle. The structure for preventing re-exposure may include a metallic spring clip or a transverse wall integrally formed with one end of the tip guard. Needle assemblies including such tip guards, however, typically include extensive mechanics for positioning of the tip guard, resulting in complex arrangements which are costly to manufacture and assemble. Also, operation of the tip guard can involve substantial manipulation by the user to extend the tip guard to a shielding position.

PCT International Publication No. WO 98/57689 discloses a shield mechanism for catheter introducer needles which includes a hinged arm mechanism for moving a tip guard along a needle to a shielding position. The hinged arm mechanism disclosed in this publication, however, includes a number of interengaging hinge mechanisms which cause the hinged arm to collapse upon itself in one position and to entirely extend or unfold to move the tip guard along the needle to the shielding position. As such, during use, the hinge arm entirely unfolds from the collapsed condition, thereby eliminating the hinged arm from the profile of the device. Moreover, the device includes complicated interengaging structure which can be difficult to manufacture and assemble.

While the above described devices provide for effective shielding of used needles, a need exists for a needle assembly for use with a blood collection set which achieves secure and effective shielding of a used needle tip and which is simple and inexpensive to manufacture and easy to operate.

### SUMMARY OF THE INVENTION

The present invention is directed to a shieldable needle device, particularly useful in connection with a blood collection set. The needle device includes a needle cannula having opposed proximal and distal ends and a lumen extending therebetween. The needle device further includes a hub having a proximal end, a distal end, and a passage extending between the ends. The proximal end of the needle cannula is securely mounted in the passage of the hub. A flexible tube may be mounted to the proximal end of the hub, such that the passage through the tube communicates with the lumen of the needle cannula. A fixture may be mounted to the end of the tube remote from the hub. The fixture enables the needle cannula and the tube to be placed in communication with an appropriate receptacle, such as a blood collection tube.

The needle device further includes a shield assembly, such as an end cap having a blocking surface that is slidably telescoped on the needle cannula for axial movement from a proximal position substantially adjacent the hub at the proximal end of the needle cannula to a distal position where the end cap protectively surrounds the distal end of the needle cannula. Additionally, the end cap may be configured to prevent proximal movement after the blocking surface of the end cap has advanced sufficiently in a distal direction to protectively enclose the distal tip of the needle assembly.

The end cap may be in the form of a tip guard, which may include a tip guard housing formed from a plastic material, and a protective clip, such as a metallic spring clip mounted to the housing. The clip is biased against the needle cannula when the tip guard is in the proximal position and is resiliently moved over the distal end and distal tip of the needle cannula when the tip guard is in the distal position, thereby preventing piercing by the tip of the needle.

The needle device further includes a guard drive for moving the tip guard from the proximal position to the distal position. The guard drive includes a rigid fin having a first end pivotally connected to the hub and an opposed second end extending dorsally from the hub. The fin is pivotally movable between a proximal position and a distal position toward the distal end of the needle cannula. The guard drive further includes a tether extending between the second end of the fin and the tip guard. The fin and the tether are adapted for corresponding movement such that pivotal movement of the fin with respect to the hub from the proximal position to the distal position causes the tether to extend toward the distal end of the needle cannula for movement of the tip guard from the proximal position to the distal position.

The needle device may include a latch for preventing movement of the tip guard from the proximal position to the distal position. The hub may include a pair of wings extending laterally from opposing sides of the hub. Also, the fin may include a pair of concave surfaces on opposing sides thereof. The tether may be a flexible material, and is desirably connected to the fin through a hinge, such as a living hinge providing for corresponding movement between the fin and the tether. In particularly desirable embodiments, the fin is integrally formed with the tether and the hub. As such, the needle device is passive, with automatic shielding of the needle tip occurring upon release of the latch, thereby permitting movement of the tip guard from the proximal position to the distal position.

### DESCRIPTION OF THE DRAWINGS

FIG. 1 is a perspective view of a blood collection set in accordance with the present invention including a packaging cover thereon;

FIG. 2 is an exploded perspective view of the shieldable needle assembly of the blood collection set of FIG. 1;

FIG. 3 is a top plan view of the shieldable needle assembly in a retracted position;

FIG. 4 is a cross-sectional view taken along line 4-4 of FIG. 3;

FIG. 5 is a top plan view of the shieldable needle assembly in an extended shielded position;

FIG. 6 is a cross-sectional view taken along line 6-6 of FIG. 5;

FIG. 7 is a cross-sectional view of a shieldable needle assembly in an alternative embodiment shown in a retracted position;

FIG. 8 is a cross-sectional view of the shieldable needle assembly of FIG. 8 shown in an extended shielded position;

FIG. 9 is a perspective view of a guard drive and hub shown in a further alternative embodiment;

FIG. 10 is a cross-sectional view of a shieldable needle assembly in an alternative embodiment including the guard drive of FIG. 9 shown in a retracted position;

FIG. 11 is a cross-sectional view of the shieldable needle assembly in an alternative embodiment including the guard drive of FIG. 9 shown in an extended shielded position;

FIG. 12 is a perspective view of a guard drive and hub shown in yet a further alternative embodiment including an alternative latching mechanism; and

FIGS. 13A, 13B and 13C are front views of the alternative mechanism of FIG. 12 showing the latch positioning during use of the shieldable needle assembly.

### DETAILED DESCRIPTION

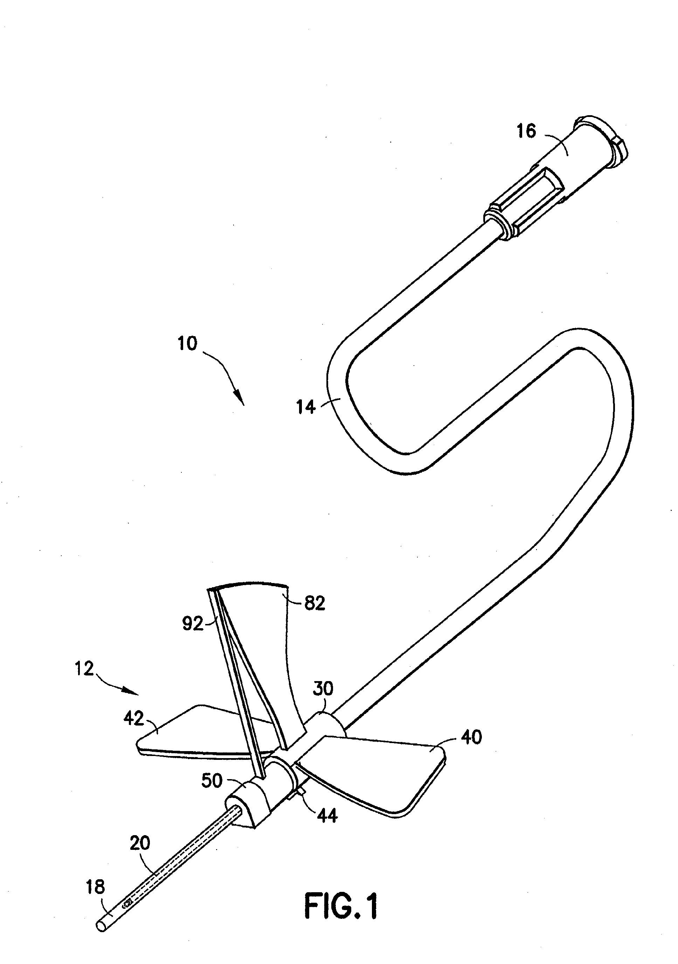

Referring to the drawings in which like reference characters refer to like parts throughout the several views thereof, FIG. 1 illustrates a blood collection set in accordance with the present invention and the related features. The present invention is generally described in terms of a blood collection set, and encompasses such a blood collection set, as well as a shieldable needle assembly for use in such a blood collection set.

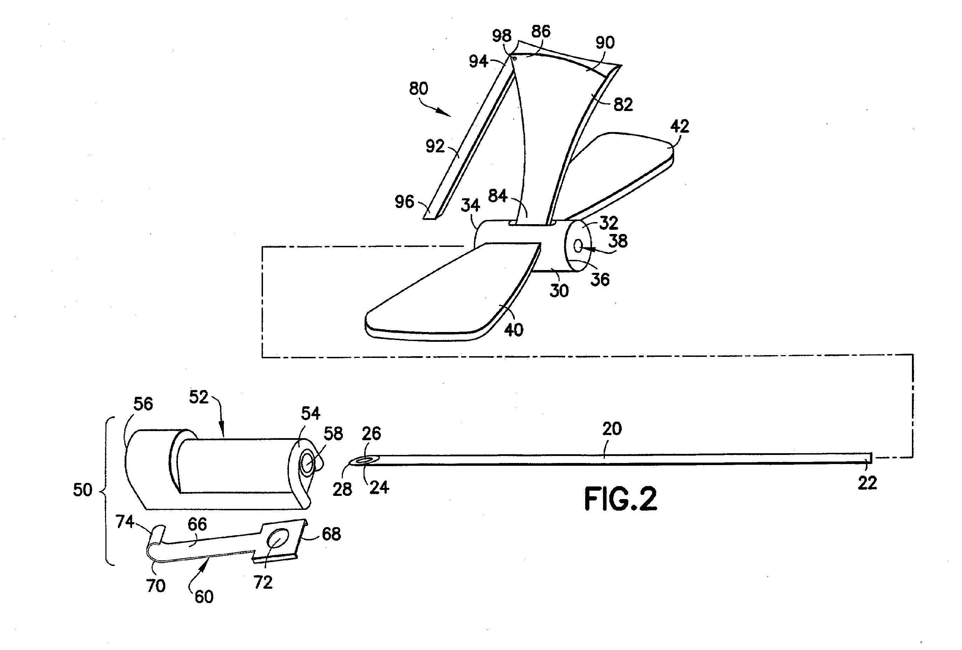

As shown in FIG. 1, blood collection set **10** includes a shieldable needle device **12**, a flexible tube **14** extending from needle device **12**, a fixture **16** mounted to tube **14**, and a packaging cover **18** removably mounted to portions of needle device **12** opposite tube **14**, such as through a frictional engagement. Shieldable needle device **12** of blood collection set **10** is shown in detail in FIGS. 2-4, and includes a needle cannula **20**, a hub **30**, a tip guard assembly **50** and a guard drive **80**, for moving the tip guard assembly.

Needle cannula **20** includes a proximal end **22** and an opposing distal end **24**, with lumen **26** extending through needle cannula **20** from proximal end **22** to distal end **24**. Distal end **24** of needle cannula **20** is beveled to define a sharp puncture tip **28**, such as an intravenous puncture tip. Puncture tip **28** is provided for insertion into a patient's blood vessel, such as a vein, and is therefore designed to provide ease of insertion and minimal discomfort during venipuncture.

Needle assembly **12** further includes hub **30**. Hub **30** is a unitary structure, desirably molded from a thermoplastic material. Hub **30** includes a proximal end **32**, a distal end **34**, and is defined by a rigid tubular wall **36** extending from proximal end **32** to distal end **34**. Tubular wall **36** is characterized by an internal passage **38** extending therethrough from proximal end **32** to distal end **34** of hub **30**. A recess **48** extends within a top portion of tubular wall **36** of hub **30**.

Hub **30** further includes a pair of stabilizers in the form of flexible wings **40** and **42** extending laterally from tubular wall **36** at opposing sides thereof. Wings **40** and **42** provide hub **30**, and needle assembly **12**, as a butterfly-type wing assembly, assistance in positioning and placement of needle assembly **12** and blood collection set **10** during a blood collection procedure, and providing a surface for securing needle device **12** to the skin of a patient, such as, through taping wings **40** and **42** to the patient's skin.

Needle cannula **20** is positioned within internal passage **38** of hub **30**, and extends from distal end **34** of hub **30**. Desirably, needle cannula **20** and hub **30** are separate parts which are fixedly attached and secured through an appropriate medical grade adhesive or the like.

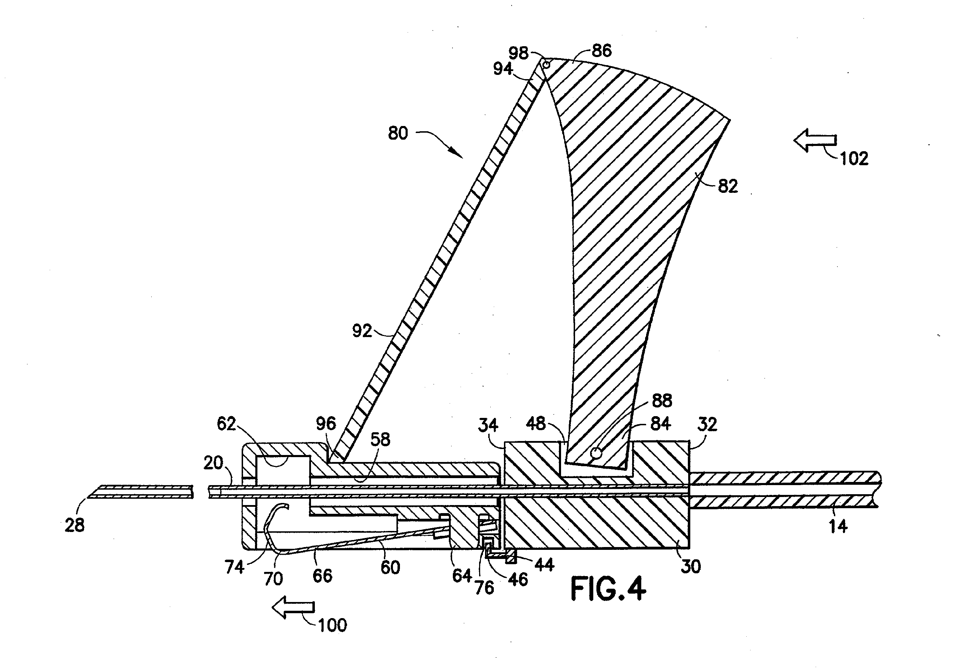

Needle assembly **12** further includes tip guard assembly **50**, which extends co-axially about needle cannula **20** and is movable along needle cannula **20** between a first proximal position adjacent hub **30**, and a second distal position adjacent puncture tip **28**, as will be described in more detail herein. Tip guard assembly **50** includes a housing **52** and a protective clip **60**. Housing **52** is a unitary structure, desirably molded from a thermoplastic material, including a proximal end **54**, a distal end **56**, and an internal passage **58** extending between the ends. Portions of internal passage **58** adjacent distal end **56** define an enlarged clip receptacle **62**, as shown in FIG. 4. A clip mounting post **64** extends downwardly from housing **52** at a location near proximal end **54** of housing **52**.

Clip **60** is unitarily stamped and formed from a resiliently deflectable metallic material. Clip **60** includes a planar spring leg **66** with a proximal end **68** and an opposed distal end **70**. A mounting aperture **72** extends through spring leg **66** at a location near proximal end 68. Mounting aperture **72** has a diameter approximately equal to or slightly less than the diameter of mounting post **64** of housing **52**. As such, mounting post **64** can be forced through mounting aperture **72** when the axis of mounting post **64** and the axis of mounting aperture **72** are substantially collinear. A lock out leg **74** extends angularly from distal end **70** of spring leg **66**. Lock out leg **74** is bent back toward proximal end **68** of clip **60**. The bends in lock out leg **74** enable secure protective engagement with puncture tip **28** of needle cannula **20** and further enable smooth axial sliding movement of tip guard assembly **50** along needle cannula **20**, as described in further detail herein.

Hub **30** and tip guard assembly **50** are interconnected through guard drive **80**. Guard drive **80** provides for axial movement of tip guard assembly **50** along needle cannula **20** from a first proximal position adjacent hub **30** to a second distal position adjacent puncture tip **28**, as will be described in more detail herein.

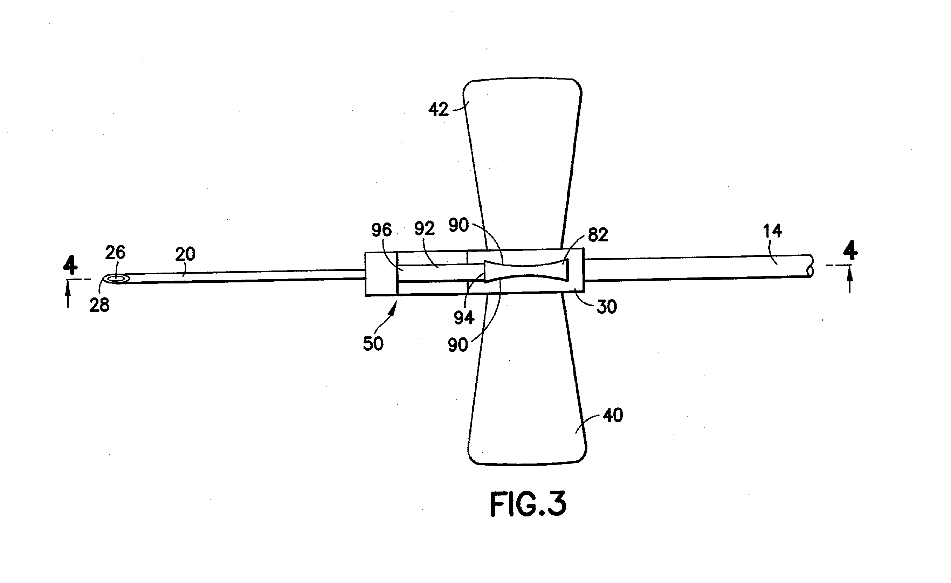

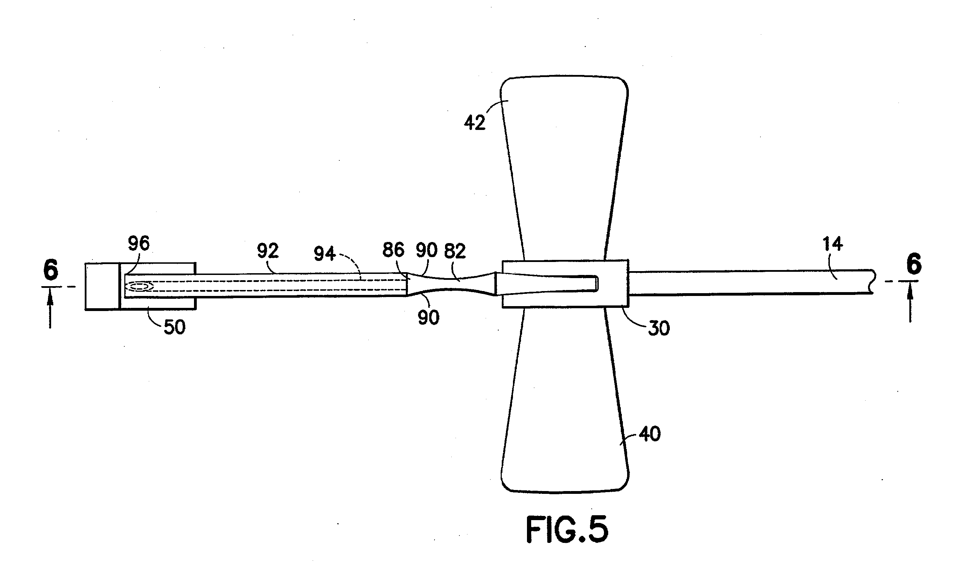

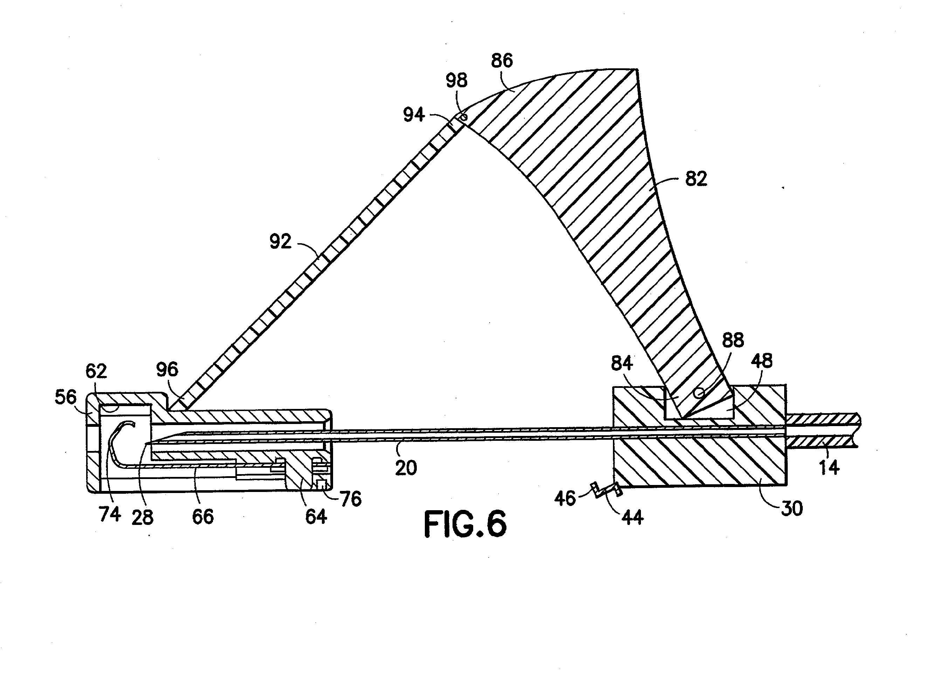

Guard drive **80** includes a fin **82** extending dorsally from a top surface of hub **30**. Fin **82** is a generally rigid structure including a first portion **84** and a second portion **86**. First portion **84** of fin **82** is pivotally connected to hub **30** for establishing pivotal movement of fin **82** with respect to hub **30**. Such pivotal movement may be established, for example, by providing first portion **84** within recess **48** at the top surface of tubular wall **36** of hub **30**, and providing a pivot **88** extending from hub **30** and through first portion **84** of fin **82** in a direction perpendicular to the general axis of needle device **12**. In this manner, fin **82** may be pivoted about an axis defined by pivot **88**, thereby causing fin **82** to move in a planar direction with respect to the general axis of needle device **12** from a proximal retracted position in which fin **82** extends dorsally from hub **30** as shown in FIGS. 3 and 4, to a distal extended position in which fin **82** remains dorsally extending from hub **30**, as shown in FIGS. 5 and 6.

As indicated, fin **82** extends from a top surface of hub **30** in a dorsal manner, that is, in a plane with the general axis of needle device **12**, providing fin **82** with opposing sides facing laterally away from a plane of symmetry. Such opposing sides desirably include concave surfaces **90**. Concave surfaces **90** are designed and configured for engagement with a thumb and index finger of a user during placement of blood collection set **10**, as will be discussed in further detail herein.

Guard drive **80** further includes a tether **92** extending between tip guard assembly **50** and second portion **86** of fin **82**. Tether **92** is desirably a resilient flexible material capable of bending and/or extending when a force is applied thereto. Tether **92** includes proximal portion **94** and distal portion **96**. Distal portion **96** of tether **92** may be fixedly attached to tip guard assembly **50**, such as through the use of an adhesive. Proximal portion **94** of tether **92** is connected to second portion **86** of fin **82**, such as through hinge **98**. Hinge **98** provides for corresponding movement between fin **82** and tether **92**, as will be discussed in more detail herein.

Since proximal portion **94** of tether **92** is connected to second portion **86** of fin **82**, pivotal movement of fin **82** about pivot **88** from the proximal position to the distal position results in corresponding movement of tether **92**. In particular, movement of fin **82** against hinge **98** exerts a biasing force against tether **92**, thereby forcing tether **92** to extend in a direction toward distal end **24** of needle cannula **20**. Moreover, since distal portion **96** of tether **92** is fixedly attached to tip guard assembly **50** and since tip guard assembly **50** is axially movable along needle cannula **20**, such biasing force against tether **92** causes tip guard assembly **50** to axially move in the direction of arrow **100** away from hub **30** and toward distal end **24** of needle cannula **20**, where tip guard assembly **50** can effectively shield puncture tip **28**.

Tip guard assembly **50** moves axially along needle cannula **20** toward distal end **24** during movement of guard drive **80** through corresponding movement between fin **82** and tether **92**. By providing hinge **98** therebetween as a living hinge, fin **82** and tether **92** can be effectively biased against each other. As such, movement of fin **82** from the proximal position to the distal position exerts a biasing force against tether **92**, thereby forcing distal portion **96** of tether **92** toward distal end **24** of needle cannula **20**. Tether **92** may also be a flexible material. As such, tether **92** may itself act as a means for storing energy to extend tether **92** toward distal end **24** of needle cannula **20** upon corresponding movement between fin **82** and tether **92**, thereby propelling tip guard assembly **50** from the proximal position to the distal position.

Needle device **12** may also be provided with means for retaining tip guard assembly **50** in the proximal position and preventing movement of tip guard assembly **50** from the proximal position adjacent hub **30** to the distal position adjacent puncture tip **28**. For example, hub **30** may include a latch **44** extending from a lower portion of tubular wall **36** adjacent distal end **34**, extending in a direction toward distal end **24** of needle cannula **20**. Latch **44** is provided for releasable engagement with tip guard assembly **50**, such as through frictional interfitting engagement between latch **44** and tip guard assembly **50**. Such interfitting engagement prevents movement of tip guard assembly **50** from the proximal position to the distal position when latch **44** is engaged with recess **76**. Desirably, latch **44** is a hinged element which includes a protrusion **46** for extending within recess **76** within the bottom of housing **52** of tip guard assembly **50**, and which is capable of hinged movement between a first position in which protrusion **46** extends within recess **76** and is therefore in frictional engagement with tip guard assembly **50**, and a second position in which protrusion **46** is out of recess **76** and therefore out of frictional engagement with tip guard assembly **50**. As such, latch **44** can be released from interfitting engagement with recess **76** of tip guard assembly **50**, thereby permitting movement of tip guard assembly **50** from the proximal position to the distal position.

It is also contemplated that hub **30** may include a latch (not shown) for preventing pivotal movement of fin **82** within recess **48** of hub **30**, in addition to or instead of latch **44** extending between hub **30** and tip guard assembly **50**.

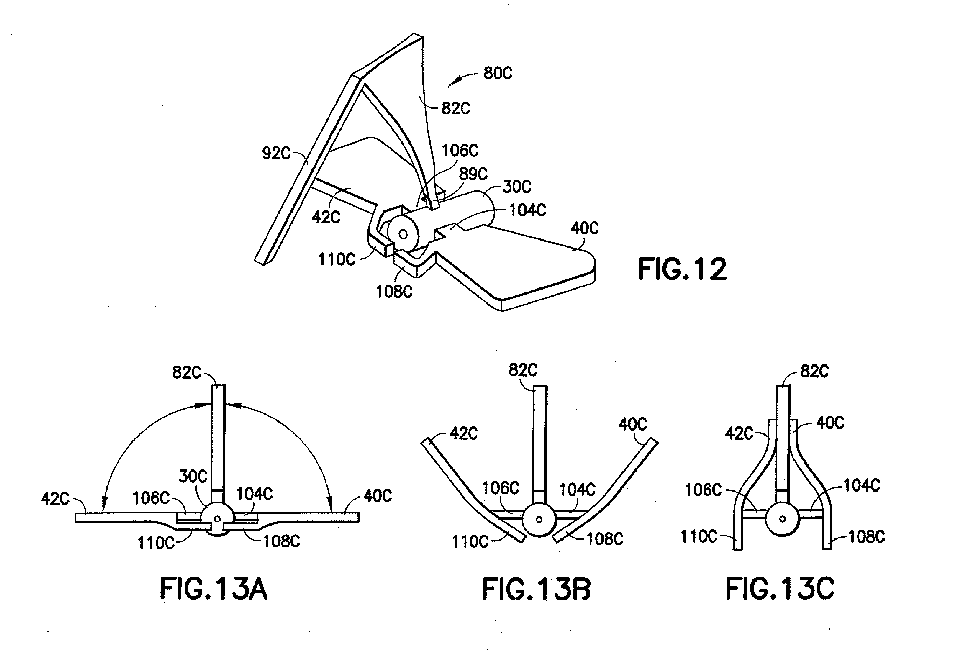

In particularly desirable embodiments, latch **44** is in corresponding engagement with at least one of wings **40** and/or **42.** For example, as noted above, hub **30** may include wings **40** and **42** extending laterally from opposing sides of tubular wall **36**. Wings **40** and **42** assist in positioning and placement of needle assembly **12** and blood collection set **10** during a blood collection procedure, and are adapted to lie flat against the surface of a patient's skin during a blood collection procedure. As such, wings **40** and **42** may be constructed of a flexible material such that at least one, and possibly both, of wings **40** and **42** can be bent toward each other and brought together between the fingers of a user to assist in positioning and placement of needle assembly **12** during venipuncture. By providing latch **44** in corresponding engagement with at least one of wings **40** and **42**, such bending of wings **40** and/or **42** will cause latch **44** to be released from interfitting engagement with recess **76** of tip guard assembly **50**, thereby permitting movement of tip guard assembly **50** from the proximal position to the distal position. Such corresponding engagement may be provided, for example, through a connection member extending between latch **44** and at least one of wings **40** and **42**, such that bending of wings **40** and **42** will cause latch **44** to release, by way of the connection member. One particular embodiment for such a release mechanism is shown in detail with reference to FIGS. 12-13C, as described herein.

Guard drive **80** may require active exertion of force thereon in order to effect movement of tip guard assembly **50** from the proximal position to the distal position in the direction of arrow **100**. For example, once latch **44** is released from engagement with tip guard assembly **50**, a force may be exerted by the user on fin **82** in the direction of arrow **102**. Such force causes fin **82** to pivotally move with respect to hub **30** about pivot **88** in a direction toward distal end **34** of hub **30** and toward distal end **24** of needle cannula **20**, i.e., between a proximal position and a distal position. Such pivotal movement causes tether **92** to extend toward distal end **24** of needle cannula **20**. Since tip guard assembly **50** is fixedly attached to tether **92**, such extension also causes movement of tip guard assembly **50** from the proximal position adjacent hub **30** to the distal position adjacent puncture tip **28**.

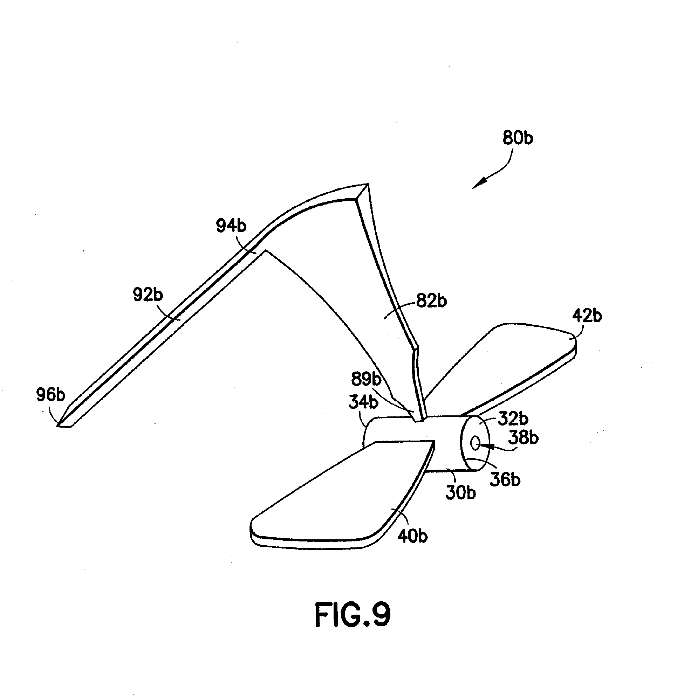

In particularly desirable embodiments, needle device **12** is passively shieldable in that it is capable of achieving secure shielding of puncture tip **28** automatically upon release of latch **44**. To achieve such passive shielding, guard drive **80** may include means for storing energy for movement of fin **82** and/or tether **92** in a direction toward distal end **24** of needle cannula **20**. For example, hinge **98** may be a torsion spring capable of exerting a biasing force upon release of engagement between hub **30** and tip guard assembly **50**, such as through latch **44**, to extend tether **92** toward distal end **24** of needle cannula **20**, thereby propelling tip guard assembly **50** from the proximal position to the distal position. Alternatively, pivot **88** may include a torsion spring capable of exerting a biasing force upon release of engagement between hub **30** and tip guard assembly **50** to move fin **82** from the proximal position to the distal position, thereby extending tether **92** toward distal end **24** of needle cannula **20** and propelling tip guard assembly **50** from the proximal position to the distal position. In a further embodiment discussed herein with reference to FIGS. 9-11, such passive shielding can be achieved, for example, by providing guard drive 80 as a unitary structure including fin **82** integrally formed with tether **92** and hub **30**, and with living hinges established therebetween which are capable of exacting biasing force to extending tether **92** and to thereby propel the tip guard assembly **50** from the proximal position to the distal position.

Tip guard assembly **50** is assembled by forcing mounting post **64** of tip guard housing **52** through mounting aperture **72** of clip **60**. Spring leg **66** of clip **60** is then urged downwardly or away from internal passage **58** through tip guard housing **52**. Distal end **22** of needle cannula **20** is then passed through internal passage **38** of hub **30**, and urged into internal passage **58** at proximal end **54** of tip guard housing **52**. The downward deflection of spring leg **66** enables distal end **24** of needle cannula **20** to be passed entirely through tip guard housing **52**. Spring leg **66** can be released after puncture tip **28** of needle cannula **20** passes entirely through tip guard housing **52**. Thus, the end of lock out leg **74** will be biased against and slide along needle cannula **20**. Tip guard assembly **50** then is slid proximally along needle cannula **20** into a position adjacent hub **30**. Packaging cover **18** is then urged over puncture tip **28** and urged proximally over needle cannula **20**, with puncture tip **28** safely maintained and disposed within packaging cover **18**.

Blood collection set **10** can be packaged substantially in the condition shown in FIG. 1. Prior to use, blood collection set **10** is removed from its package. Fixture **16** then may be connected to an appropriate receptacle for providing fluid communication with lumen **26** through needle cannula **20**.

In use, blood collection set **10** is provided with needle device **12** assembled and including flexible tube **14** extending from needle device **12** and connected to fixture **16**. After removing blood collection set **10** from its package, it can be assembled with other appropriate medical equipment for use. For example, a non-patient needle assembly and a needle holder may be connected to blood collection set **10** through fixture **16**.

To prepare for use of blood collection set **10**, the user grasps blood collection set **10** at needle device **12**, placing a thumb and forefinger between one concave surface **90** of fin **82** and one of wings **40** or **42** of hub **30**, with fin **82** maintained between the user's fingers. Alternatively, both wings **40** and **42** can be flexed or bent toward each other between a user's thumb and forefinger with fin **82** trapped therebetween. Fin **82** may be of sufficient length to extend dorsally beyond wings **40** and **42** when wings **40** and **42** are flexed or bent together, thereby providing a further surface for grasping between the user's thumb and forefinger. Packaging cover **18** is then grasped and urged distally to disengage from needle cannula **20**, thereby exposing puncture tip **28** of needle cannula **20**.

The medical practitioner can then urge puncture tip **28** at distal end **24** of needle cannula **20** into a targeted blood vessel of a patient, while guard drive **80** is maintained between thumb and forefinger to assist in controlled entry by the medical practitioner. During such positioning, at least one of wings **40** and **42** is bent inwardly toward the other and toward fin **82** between the user's fingers. Such bending causes latch **44** to disengage from engagement with tip guard assembly **50**. Alternately, latch **44** may be manually released by the user's finger. In embodiments incorporating a torsion spring or other biasing means as described above, tip guard assembly **50** is maintained in the proximal position due to the grip by the user's fingers between one of wings **40** or **42** and the fin **82** at concave surface **90,** even though latch **44** is released. As such, fin **82** is prevented from movement from the proximal position to the distal position due to the user's grasp against concave surface **90.**

After the targeted blood vessel has been accessed, the medical practitioner can release the grip on guard drive **80**. Once the user releases the device, fin **82** is free to pivotally move from the proximal position to the distal position, due to the bias exerted between tether **92** and fin **82** through hinge **98**. Such movement causes tether **92** to extend, thereby propelling tip guard assembly **50** distally along needle cannula **20** in an axial direction of arrow **100**, with tip guard assembly **50** sliding or gliding along needle cannula **20** toward distal end **24**. Distal movement of tip guard assembly **50** will terminate when proximal end **54** of tip guard housing **52** contacts the skin of the patient near the puncture site.

Upon completion of the procedure, such as when all desired samples have been drawn, needle cannula **20** is withdrawn from the patient. This removal of needle cannula **20** from the patient will permit further extension of tether **92** and a corresponding distal movement of tip guard assembly **50** in an axial direction of arrow **100**. After tip guard assembly **50** is moved along needle cannula 20 to the distal end **24**, lockout leg **74** of clip **60** will pass distally beyond puncture tip **28** of needle cannula **20**. The inherent resiliency of spring leg **66** of clip **60** will urge lockout leg **74** over puncture tip **28** of needle cannula **20**. Thus, a return movement of tip guard assembly **50** is prevented. Furthermore, guard drive **80** has an overall dimension that will prevent movement of tip guard assembly **50** distally beyond needle cannula **20**. Hence, puncture tip **28** of needle cannula **20** is safely shielded. Blood collection set **10** may then be appropriately discarded.

Since fin **82** of guard drive **80** extends dorsally from hub **30**, fin **82** can act as a handle portion during insertion, withdrawal and disposal of needle device **12**. In particular, even after activation of the shielding feature in order to propel tip guard assembly **50** to the distal position shielding needle cannula **20**, fin **82** is maintained in a dorsal position, albeit at a slightly forward or distal position. Since fin **82** still extends dorsally and is a rigid structure, fin **82** can be used to grip needle device **12** after removal from the patient, and can act as a handle portion for carrying blood collection set **10** at a position remote from the used needle tip of cannula **20**.

It is noted that while activation of the safety feature may be automatically accomplished upon release of latch **44**, providing needle device **12** with a passively shielding feature, it is also contemplated that activation of the shielding feature may require a specific force exerted by the user in a direction of arrow **102**, as discussed above. In such situations, guard drive **80** can be activated while puncture tip **28** is within the patient's blood vessel, thereby axially moving tip guard assembly **50**, axially along needle cannula **20**, or may be activated after puncture tip **28** is removed from the patient's blood vessel.

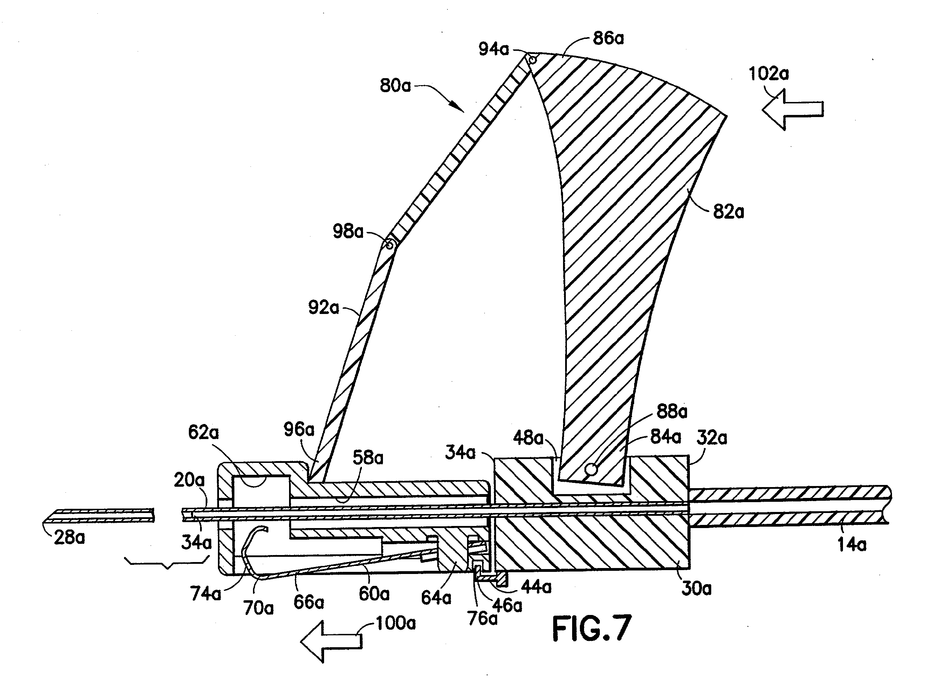

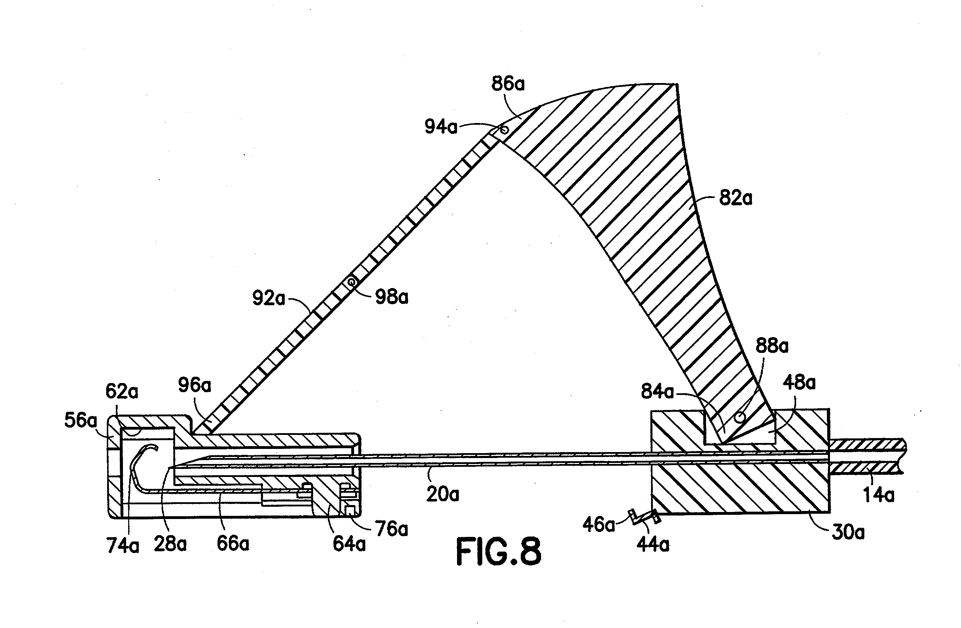

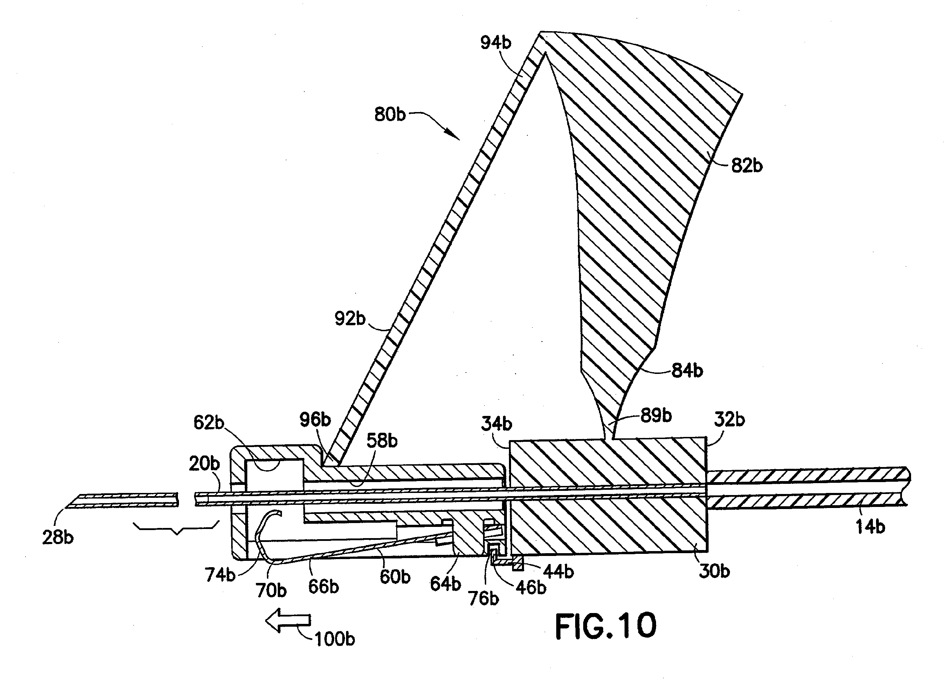

FIGS. 7-13 depict further embodiments of the present invention that include many components which are substantially identical to the components of FIGS. 1-6. Accordingly, similar components performing similar functions will be numbered identically to those components of FIGS. 1-6, except that a suffix "a" will be used to identify those similar components in FIGS. 7 and 8, a suffix "b" will be used to identify those similar components in FIGS. 9- 11, and a suffix "c" will be used to identify those similar components in FIGS. 12 and 13A-C.

In a further embodiment depicted in FIGS. 7 and 8, the tether **92a** includes two sections that meet at a hinge **98a**. In particular, guard drive **80a** includes tether **92a** including proximal portion **94a** and distal portion **96a**. Proximal portion **94a** is fixedly adhered to fin **82a** at second portion **86a** of fin **82a**, desirably in a flexing joint. Distal portion **96a** is fixedly adhered to tip guard assembly **50a** as in the embodiment described above with reference to FIGS. 1-6. Proximal portion may be a rigid material, and is preferably constructed of a similar material as fin **82a**. In particularly preferred embodiments, proximal portion **94a** is integrally formed with fin **82a** as an extension thereof with the juncture between proximal portion **94a** and fin **82a** acting as a living hinge. Distal portion **96a** may be a bendable or flexible material, capable of extending.

Proximal portion **94a** and distal portion **96a** are interconnected through hinge **98a**. Hinge **98a** may be a living hinge, but is desirably a torsion spring capable of exerting a biasing force upon release of engagement between hub **30a** and tip guard assembly **50a** to extend distal portion **96a** of tether **92a** toward distal end **24a** of needle cannula **20a**, thereby propelling tip guard assembly **50a** from the proximal position to the distal position. As such, needle device **12a** is provided with a passive shielding feature.

In a further embodiment depicted in FIGS. 9-11, guard drive **80b** is integrally formed with hub **30b** and tether **92b**, forming a one-piece unitary structure. In particular, guard drive **80b** includes a fin **82b** extending dorsally from and pivotal with respect to a top surface of hub **30b** in a similar manner as in the embodiment described with respect to FIGS. 1-6. Pivotal movement of guard drive **80b**, however, is established through the specific structure of hub **30b** and fin **82b** which creates a living hinge **89b** for pivotal movement therebetween. Desirably, the structure of fin **82b** is cut away on the opposing sides thereof to form a narrow portion at first end **84b** at the point of connection with hub **30b**. This narrowing portion allows for flexibility between fin **82b** and hub **30b**, with the wall portion of fin **82b** at this narrowing portion acting as a living hinge for pivoting movement of fin **82b**.

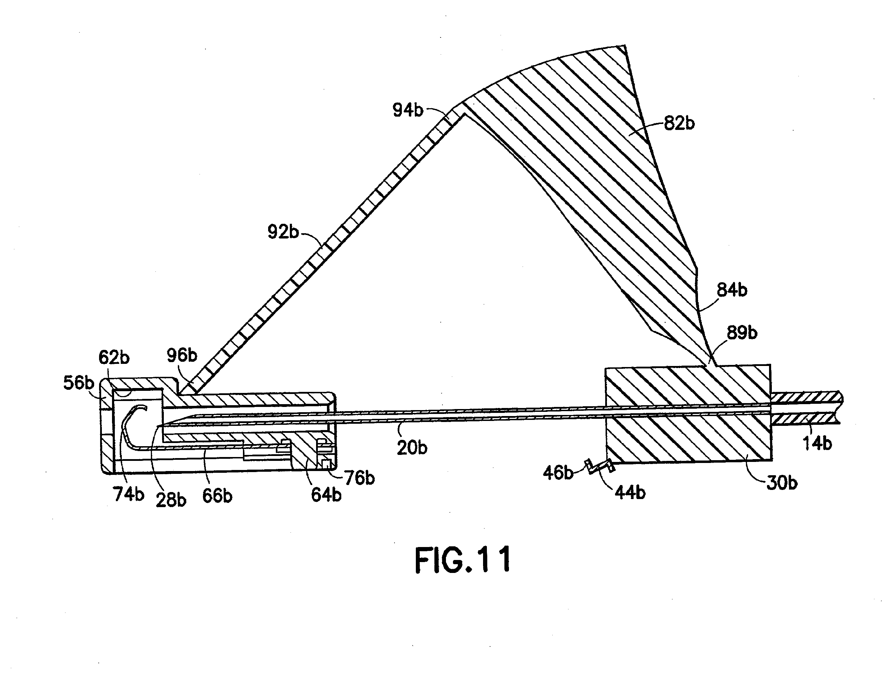

As noted, guard drive **80b** is desirably integrally formed with tether **92b** and hub **30b**. Desirably, guard drive **80b** is an integral structure which is in a relaxed state with fin **82b** in a distal position and with tether **92b** extended toward the distal position, as shown in FIGS. 9 and 11. As such, the natural tendency for guard drive **80b** is to return to this relaxed state with fin **82b** in a distal position and with tether **92b** extended toward the distal position. In such an embodiment, the device may be assembled and packaged with guard drive **80b** retained in the proximal position as shown in FIG. 10, such that guard drive **80b** is biased against its natural relaxed state. In this manner, guard drive **80b** is effectively "primed" for operation to propel the tip guard to a distal position encompassing puncture tip **28b** of needle cannula **20b**. Guard drive **80b** may be retained in this proximal position against its natural bias through any mechanism, such as through friction established by a packaging cover covering needle cannula **20b**, or through a latch **44b** as described in connection with the embodiment described above.

It is further contemplated that guard drive **80b** is in a relaxed stated during packaging. Prior to use, guard drive **80b** can be pivoted to the proximal position and held in such a position between wings **40b** and **42b** held between a user's finger and thumb such that guard drive **80b** is biased against its natural relaxed stated and is primed for operation. Upon release, guard drive **80b** returns to its natural unbiased stated, and therefore propels the tip guard to a distal position encompassing puncture tip **28b** of needle cannula **20b**.

As described above, in embodiments incorporating biasing means for providing a passively shielding feature, such as by providing hinge **98a** as a torsion spring or by providing guard drive **80b** with a living hinge **89b** which is biased against a relaxed state, passive shielding of the needle cannula is automatically achieved merely by removing needle cannula from the patient. In some instances, however, the needle device may be dropped or knocked from the hand of the medical practitioner either before venipuncture or during a medical procedure. The passive shielding described above will commence automatically when the needle device is dropped or knocked from the medical practitioner's hand. Thus, the automatic shielding may be triggered by the intentional or unintentional release of the fin by the medical practitioner.

Moreover, a medical practitioner does not always enter the targeted blood vessel during the first venipuncture attempt. However, a medical practitioner typically retains a close grip on the needle device until the targeted blood vessel has been entered. In this instance, the continued gripping between the fin and the wings will prevent the needle from shielding until the targeted blood vessel has been punctured. The second attempt at accessing a targeted blood vessel generally is a very low risk procedure in which the practitioner's hand is spaced considerably from the puncture tip of the needle cannula. Thus, the blood collection set according to the present invention does not involve the inconvenience of having to use a new blood collection set following each unsuccessful venipuncture attempt.

As noted above, FIGS. 12-13C depict a further embodiment of the present invention which includes an alternate latch mechanism for retaining the tip guard assembly in the proximal position adjacent hub **30c** and preventing movement of the tip guard assembly until use. In particular, the embodiment shown in FIGS. 12-13C demonstrates a latch mechanism which is in corresponding engagement with at least one, and desirably both of the flexible wings extending laterally from the hub, as described above in connection with the embodiment shown in FIGS. 1-6.

In the embodiment of FIGS. 12-13C, wings **40c** and **42c** extend laterally from opposing sides of hub **30c** and are interconnected therewith through rigid struts **104c** and **106c**, respectively. Rigid struts **104c** and **106c** are desirably constructed of the same material as hub **30c**, and are rigid members which are not meant to flex, or are meant to flex only slightly during bending of wings **40c** and **42c**. As such, rigid struts **104c** and **106c** act in effect as fulcroms for flexing of wings **40c** and **42c** during use. Alternatively, wings **40c** and **42c** may include a rigid portion at the point of attachment with hub **30c**, thereby eliminating the need for any such rigid struts **104c** and **106c**.

Wings **40c** and **42c** include latches **108c** and **110c**, respectively, which extend from the forward or distal edges thereof radially inward toward the distal end **34c** of hub **30c**. Latches **108c** and **110c** are configured for releasable interfitting engagement with a recessed portion of the tip guard assembly, such as recess **76** of tip guard assembly **50**, described above and depicted in FIG. 4. Desirably, such a recessed portion is in the form of an annular ring extending about at least a proximal portion of the housing of the tip guard assembly, such that latches **108c** and **110c** extend within such an annular ring when wings **40c** and **42c** are in their natural state extending laterally from opposing sides of hub **30c** in a planar manner. In this manner, latches **108c** and **110c** are in interference engagement with the tip guard assembly and therefore retain the tip guard assembly at a proximal position adjacent hub **30c**.

During use, wings **40c** and **42c** are bent inwardly toward each other and toward fin **82c**, to assist in positioning and placement of the needle assembly during a procedure, as described above. During such bending, rigid struts **104c** and **106c** act as fulcroms for bending of wings **40c** and **42c**, as shown in sequence in FIGS. 13A-13C. In this manner, latches **108c** and **110c** in turn are moved or pivoted out of interference engagement with the recess of the tip guard assembly. In particularly desirable embodiments which incorporate a biasing means for biasing the guard drive forward toward the distal position for shielding of the needle such as the guard drive **80b** including living hinge **89b** described in connection with FIGS. 9-11, release of latches **108c** and **110c** will cause the tip guard assembly to be released from its retracted proximal position. As shown in FIG. 13C, however, when wings **40c** and **42c** are in a position to release latches **108c** and **110c**, wings **40c** and **42c** are bent against fin **82c** of guard drive **80c**, for example between a user's finger and thumb. As such, the pressure between wings **40c** and **42c** between a user's finger and thumb exerts a normal force between wings **40c** and/or **42c** and guard drive **80c**, thereby retaining fin **82c** in the proximal dorsal position, thus preventing movement of fin **82c** to the distal dorsal position which would cause activation of guard drive **80c** to propel the tip guard assembly distally forward. As shown in FIG. 13C, ideally fin **82c** has a sufficient dorsal length when measured from hub **30c** to allow for a dorsal portion of fin **82c** to remain exposed in relation to wings **40c** and **42c** when the wings are moved towards adjacent alignment with fin **82c**. This provides the user with the ability to hold fin **82c** in its proximal dorsal position between a user's finger and thumb and have sufficient clearance to move wings **40c** and **42c** in and out of a position bent against fin **82c**, and where a user could re-grip fin **82c** along with wings **40c** and **42c**.

Once the needle assembly is properly placed at the intended site, the user can release the grip between wings **40c** and **42c**, which will release fin **82c**, and in turn will release guard drive **80c** for activation, at which time the tip guard assembly will move forward to a position against the patient's skin until the needle is removed from the patient, at which time the tip guard assembly with automatically and passively shield the needle tip. In this manner, the needle assembly is passively activated during a normal sequence of operation and use, in that the typical operation of bending of the wings for positioning and placement of the needle causes release of the tip guard assembly, and the same motion for bending of the wings also retains the thus released tip guard assembly in the retracted position until the needle assembly is released from the user's grip.

While the needle assembly of the present invention has been described in terms of one embodiment for use in connection with a blood collection system, it is further contemplated that the needle assembly could be used with other medical procedures, such as in conjunction with a conventional intravenous infusion set, which are well known in the art for use with needle assemblies.

While the present invention is satisfied by embodiments in many different forms, there is shown in the drawings and described herein in detail, the preferred embodiments of the invention, with the understanding that the present disclosure is to be considered as exemplary of the principles of the invention and is not intended to limit the invention to the embodiments illustrated. Various other embodiments will be apparent to and readily made by those skilled in the art without departing from the scope and spirit of the invention. The scope of the invention will be measured by the appended claims and their equivalents.

## Claims

1. A shieldable needle device comprising:
a needle cannula having opposed proximal and distal ends;
a hub mounted to said proximal end of said needle cannula;
a tip guard axially movable along said needle cannula from a proximal position substantially adjacent said hub to a distal position where said tip guard protectively surrounds said distal end of said needle cannula; and
a guard drive for moving said tip guard from said proximal position to said distal position, said guard drive comprising a rigid fin having a first end pivotally connected to said hub and an opposed second end extending dorsally from said hub and movable from a proximal dorsal position to a distal dorsal position, said guard drive further comprising a tether extending between said second end of said fin and said tip guard, said fin and said tether adapted for corresponding movement such that pivotal movement of said fin with respect to said hub from said proximal dorsal position to said distal dorsal position causes said tether to extend toward said distal end of said needle cannula for movement of said tip guard from said proximal position to said distal position.

2. A needle device as in claim 1, further comprising a latch for preventing movement of said tip guard from said proximal position to said distal position.

3. A needle device as in claim 1, wherein said hub includes a pair of wings extending laterally from opposing sides of said hub.

4. A needle device as in claim 1, wherein said fin includes a pair of concave surfaces on opposing sides thereof.

5. A needle device as in claim 1, wherein said tether is connected to said second end of said fin through a hinge, said hinge providing said corresponding movement between said fin and said tether.

6. A needle device as in claim 1, wherein said fin is integrally formed with said hub and said tether, thereby establishing a first living hinge between said first end of said fin and said hub and a second living hinge between said second end of said fin and said tether.

7. A needle device as in claim 6, wherein said guard drive is in a biased state when said fin is in said proximal dorsal position.

8. A needle device as in claim 1, wherein said tip guard comprises a tip guard housing formed from a plastic material, a metallic spring clip being mounted to said housing, said spring clip being biased against said needle cannula when said tip guard is in said proximal position and being resiliently moved over said distal end of said needle cannula when said tip guard is in said distal position.

9. A needle device as in claim 1, wherein said hub is adapted for connection to a flexible tube of a blood collection set.

10. A shieldable needle device comprising:
a needle cannula having opposed proximal and distal ends;
a hub mounted to said proximal end of said needle cannula, said hub including a rigid fin having a first end pivotally connected to said hub and an opposed second end extending dorsally from said hub, said fin pivotally movable between a dorsally extending proximal position and a dorsally extending distal position toward said distal end of said needle cannula;
a tip guard axially movable along said needle cannula from a proximal position substantially adjacent said hub to a distal position where said tip guard protectively surrounds said distal end of said needle cannula; and
a resilient flexible tether connected with said second end of said fin and extending between said fin and said tip guard,
wherein pivotal movement of said fin from said dorsally extending proximal position to said dorsally extending distal position causes said tether to extend toward said distal end of said needle cannula for movement of said tip guard from said proximal position to said distal position.

11. A needle device as in claim 10, further comprising a latch for preventing movement of said tip guard from said proximal position to said distal position.

12. A needle device as in claim 10, wherein said hub further includes a pair of wings extending laterally from opposing sides of said hub.

13. A shieldable blood collection set comprising:
a flexible tube having opposed first and second ends, said first end of said flexible tube being adapted for connection to a blood collection receptacle;
a hub mounted to said second end of said flexible tube;
a needle cannula having a proximal end connected to said hub, a distal end projecting from said hub and a lumen in fluid communication with said flexible tube and said fixture;
a tip guard axially movable along said needle cannula from a proximal position substantially adjacent said hub to a distal position surrounding said distal end of said needle cannula; and
a guard drive for moving said tip guard from said proximal position to said distal position, said guard drive comprising a rigid fin having a first end pivotally connected to said hub and an opposed second end extending dorsally from said hub and movable from a proximal dorsal position to a distal dorsal position, said guard drive further comprising a tether extending between said second end of said fin and said tip guard, said fin and said tether adapted for corresponding movement such that pivotal movement of said fin with respect to said hub from said proximal dorsal position to said distal dorsal position causes said tether to extend toward said distal end of said needle cannula for movement of said tip guard from said proximal position to said distal position.

14. A blood collection set as in claim 13, further comprising a packaging cover frictionally engaged on said needle cannula and securely surrounding said needle cannula.

15. A blood collection set as in claim 13, wherein said tip guard comprises a rigid housing having an aperture extending therethrough, said needle cannula being slidably disposed in said aperture, said tip guard further comprising a metallic clip mounted to said housing and configured for sliding engagement against said needle cannula as said tip guard moves from said proximal position toward said distal position, said metallic clip being dimensioned and disposed to protectively cover said distal end of said needle cannula when said tip guard has reached said distal position.

16. A blood collection set as in claim 13, further comprising a latch for preventing movement of said tip guard from said proximal position to said distal position.

17. A blood collection set as in claim 13, wherein said hub includes a pair of wings extending laterally from opposing sides of said hub.

18. A blood collection set as in claim 13, wherein said fin includes a pair of concave surfaces on opposing sides thereof.

19. A blood collection set as in claim 13, wherein said fin is integrally formed with said hub and said tether, thereby establishing a first living hinge between said first end of said fin and said hub and a second living hinge between said second end of said fin and said tether.

20. A blood collection set as in claim 19, wherein said guard drive is in a biased state when said fin is in said proximal dorsal position.

21. A shieldable needle device comprising:
a needle cannula having opposed proximal and distal ends;
a tip guard axially movable along said needle cannula from a proximal position substantially adjacent said proximal end of said needle cannula to a distal position where said tip guard protectively surrounds said distal end of said needle cannula; and
a hub mounted to said proximal end of said needle cannula, said hub including a pair of flexible wings extending laterally from opposing sides of said hub with at least one of said wings including a latch in engagement with said tip guard for maintaining said tip guard in said proximal position, said hub further including a guard drive comprising a rigid fin having a first end pivotally connected to said hub and an opposed second end extending dorsally from said hub and pivotal between a dorsally extending proximal position in which said guard drive is in a biased state and a dorsally extending distal position toward said distal end of said needle cannula, said guard drive further comprising a tether extending from said second end of said fin and connected with said tip guard,
wherein bending of said flexible wings toward said fin releases said latch from engagement with said tip guard and maintains said fin in said proximal position with said guard drive in a biased state, and flexing of said flexible wings away from said fin releases said fin so as to bias said guard drive toward said distal position, thereby causing said tether to extend toward said distal end of said needle cannula for movement of said tip guard from said proximal position to said distal position.

22. A needle device as in claim 21, wherein both of said pair of flexible wings includes a latch in engagement with said tip guard.

23. A needle device as in claim 21, wherein said pair of flexible wings are attached to said hub through a rigid strut.

24. A blood collection set as in claim 19, wherein said guard drive is in a biased state when said fin is in said proximal dorsal position.
